# EUROPEAN PATENT APPLICATION

(11) **EP 4 813 371 A1**
(43) Date of publication of application: **30.09.2026**
(21) Application number: 26167021.0
(22) Date of filing: 24.03.2026
(51) Int. Cl.: A61K 8/92, A61Q 13/00

(54) **MANUFACTURING PROCESS OF COSMETIC AND/OR HOME CARE PRODUCTS, UNDERWATER APPARATUS FOR SUCH PROCESS AND PRODUCT SO OBTAINED**

(30) Priority: 24.03.2025 IT 202500005961
(71) Applicant: SDF Group Company di Saira de Ferrari Ditta individuale, 16123 Genova (IT)
(72) Inventor: DE FERRARI, Saira, GENOVA (IT)
(74) Representative: Metroconsult Srl

(57) **Abstract**

The invention relates to a method for the production of cosmetic or home care products, wherein a perfume, an essence or a composition thereof with an aqueous, alcoholic, hydroalcoholic or oily base is subjected to a phase of refining and/or aging, stabilization in bottles, flasks (10). According to the invention, said refining phase is carried out by immersing the bottles (10) in water, preferably sea water. The invention also includes a case for immersing the bottles in the sea, and the product obtained.

## Description

The invention generally relates to the production of cosmetics, meaning by this term the substances, or mixtures of substances, intended to be applied externally to the parts of the human body, for example on the epidermis, hair, nails, lips or others, for the main purpose of cleaning, perfuming, modifying their appearance, protecting, toning or keeping them in good condition.

Although many products fall within the scope of this definition, such as soaps, lotions, bath salts, creams of various types, powders, makeup, enamels, emulsions, lotions, gels, oils, talc, etc., the present invention is intended in particular for products comprising an aqueous, alcoholic, hydroalcoholic, or oily base.

In the remainder of this description, general reference will be made to cosmetics to indicate the products mentioned above, even though the principles of the invention can be applied to different products that have some analogies with them, such as home care products.

As is known, cosmetic products are generally composed of one or more active ingredients mixed with a "base," which in turn is made up of emulsifiers, preservatives, antioxidants, humectants, silicones, colorants, perfumed essences, etc.

The chemical-physical composition of cosmetics and the combination of the various elements with which they are made is subject to regulations by national or international bodies responsible for ensuring their compatibility with people's health; an example of these bodies is represented by INCI (International Nomenclature of Cosmetic Ingredients), which is the international system for classifying any natural or synthetic element present in cosmetics.

The production processes vary depending on the cosmetics to be made; they can take place either on an industrial scale and therefore with large quantities of raw materials processed by apparatuses in production lines, or on a smaller or artisanal scale by working and/or combining the various ingredients in a laboratory, possibly also made by third-party producers or suppliers, following recipes and protocols developed by the manufacturer who makes the final product. Regardless of the manufacturing method and the type of cosmetic to be obtained, in the prior art there are known production processes in which intermediate or final compositions are subjected to one or more phases including refining, aging, seasoning, stabilization, mixing, infusion, maturation, maceration or similar, during which the product is kept in controlled environmental conditions and for a time adequate to reach the desired properties.

The controlled conditions can concern various process parameters such as temperature, light radiation, humidity, pressure, agitation and anything else, depending on the substances or compositions of substances to be processed.

For example, in the case of compositions with an alcoholic, aqueous, hydroalcoholic, or oily base, the stabilization of the latter with odorous essences or other substances such as watersoluble or fat-soluble ones used in cosmetics or home care, preferably occurs over quite long times (days, weeks or more) to allow the components to go into solution or to mix or stabilize. Sometimes it is also necessary to provide some agitation to the compositions being processed, and for this reason, suitable equipment is used for this purpose.

Although this way of operating is now consolidated and established in the state of the art, it nevertheless has some contraindications and limitations.

Firstly, it should be noted that maintaining the products in controlled conditions requires the continuous operation of the designated equipment; since the latter is equipped with the necessary means to regulate temperature, humidity, agitation and so on, it follows that in addition to the energy consumption, which can be high for long work cycles, it is also necessary to provide suitable systems to ensure continuous operation (e.g., batteries or backup power generators).

Consequently, production costs can be high also due to the equipment, which is expensive, especially when referring to artisanal productions as in small and medium-sized companies. Furthermore, given that energy consumption can be non-negligible, the known production processes are also underperforming from the point of view of energy and environmental sustainability.

Moreover, even on a large industrial scale, the known production processes are not optimal, as they involve a production modality by prepared lots (so-called "batch") that depend on the capacity of the machines.

In other words, if it is requested to increase the production capacity at an industrial level, it is necessary to proportionally increase the number of machines and/or their dimensions, and therefore in this case too, economic investments are necessary, which can be onerous with all the negative consequences deriving thereto.

From the energy and environmental sustainability point of view, the same drawbacks already mentioned remain, so that even under this profile the prior art is not satisfactory.

In light of these assumptions, the applicants aim at improving the prior art relating to the production of cosmetic and/or home care products.

In particular, the technical problem underlying the present invention is to provide a manufacturing method for the production of compositions, in particular with an aqueous, alcoholic, hydroalcoholic, or oily base, in which the resting phase in controlled conditions of the products occurs in a generally sustainable way and, above all, from an economic and energy point of view.

The idea for solving this problem is to carry out said resting phase under controlled conditions in a natural way, thereby avoiding use of electrical power for the operation of electrical equipment.

Preferably, said phase is carried out in a natural underwater environment such as the marine environment, at a depth where the temperature, pressure, luminosity and/or other parameters (e.g. salinity), are suitable for the permanence of the substances in the desired conditions.

To this end, the substances are placed in special containers which, in turn, are advantageously housed in a support structure, that can be lowered into the water and maintained at the required depth.

Preferably, the structure is configured in a substantially open manner, so that it can be passed through by water flow which, by interacting with the containers, produces a natural agitation effect.

The features of the production process according to the invention are stated more specifically in the appended claims.

The invention also includes means for carrying out the process, whose features are also stated in the claims.

Such characteristics, the effects deriving therefrom and the advantages achieved by the invention will become more apparent from the following description of a preferred and non-exclusive embodiment thereof, with reference to the attached drawings wherein:
- Fig. 1 is a flowchart illustrating the operating phases of an example of a manufacturing process of cosmetic products, in accordance with the invention;
- Fig. 2 is a schematic representation of a phase of the process of the invention;

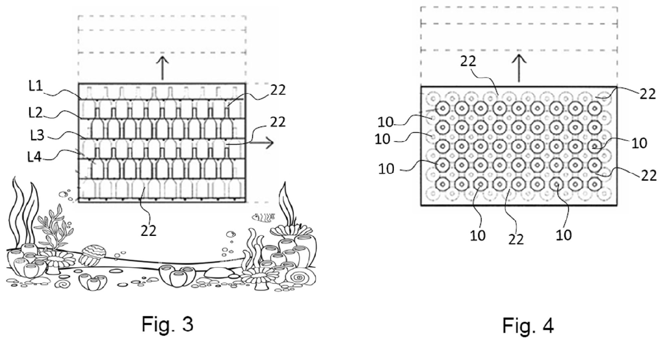

- Fig. 3 and 4 are respective front and bottom views of a case for housing the products in a phase of the process according to the invention;
- Fig. 5 shows an embodiment of a bottle or flask for cosmetic products, applied in the process of the invention;
- Fig. 6 is a flowchart illustrating the phases of a variant of the process of fig. 1.

With reference to the figures considered herein and specially to the first of them, an example of a process for producing cosmetics in accordance with the invention provides for a perfume or a natural essence to be initially manufactured by mixing the various ingredients according to the formulations required by the producer (Phase I).

The product obtained is then matured into suitable containers, preferably appropriate for maintaining controlled pressure and temperature conditions, as well as preventing contact with the external environment and the dispersion of volatile substances (Phase II).

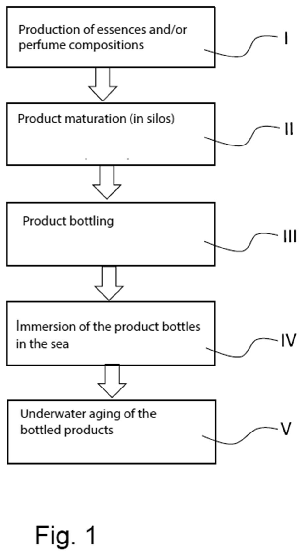

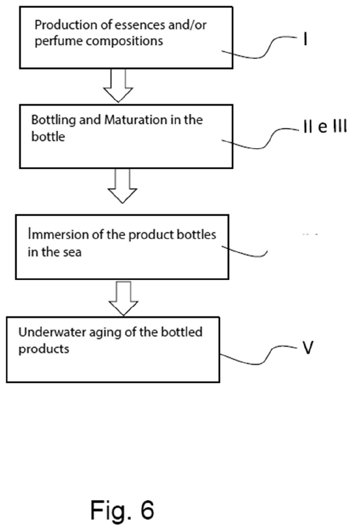

Sometimes the maturation phase II can be completed during the subsequent production phases, as for example visible in figure 6 which shows a variant of the process of fig. 1, wherein the maturation phase II occurs at least in part in the bottles.

In the flowchart, phases II and III are therefore grouped together.

For this purpose, containers such as steel silos or other materials that do not contaminate the essences being processed can be used.

As said, the cosmetic products considered here can be either concentrated essences or compositions with an alcoholic, aqueous, hydroalcoholic, or oily base, the latter preferably comprising lipophilic ester solvents.

The compositions, after being bottled (Phase III), must be refined, stabilized, or in any case finished by keeping them in appropriate conditions of temperature, light, humidity, as well as being mixed.

To this end, in accordance with the invention, the applicants found out in a surprising and original manner that suitable conditions for this production phase can be found in the marine environment where at a depth of few meters, preferably a few tens of meters, the temperature remains stable within a range of values varying from 6-9 °C to 14-16 °C throughout the year. Furthermore, the luminosity is also reduced as a function of depth, while the surrounding pressure increases; moreover, in the sea there are also currents that can be exploited to exert an agitation force on the bottles.

All of these conditions together can hardly be found on dry land and therefore, for this reason, the refining of the product according to the invention is carried out in an underwater environment (Phases IV and V).

To this end, the bottles or flasks 10 for packaging the product are preferably made of glass and closed with a cap 12 which can be of various materials, depending on the type of product being refined.

The thickness of the glass of the bottle 10 must be adequate to resist the hydrostatic pressure at the depth of the sea, while the cap and more generally the closure 12 must seal the bottles 10 without contaminating their content, in any case resisting to the submarine conditions.

For this purpose, the closure 12 can be combined, i.e., comprise multiple elements.

For example, the closure 12 can comprise a cork stopper 14, preferably ground with the addition of additives to resist salt water, together with a crown cap 15 of metal or plastic or in any case of a material resistant to prolonged permanence in water.

Alternatively, the closure 12 can be single, i.e., formed by a single element such as a crown cap of metal or plastic or in any case of a material resistant to prolonged permanence in water.

Optionally, one or the other of the above-mentioned closures 12 can be integrated with the application of a capsule (not shown in the drawings), i.e., a cover foil to protect the caps 14, 15; the capsule can be made of plastic material, optionally also heat-shrinkable, or of metal such as aluminum, copper or other appropriate metal to resist in a saline environment.

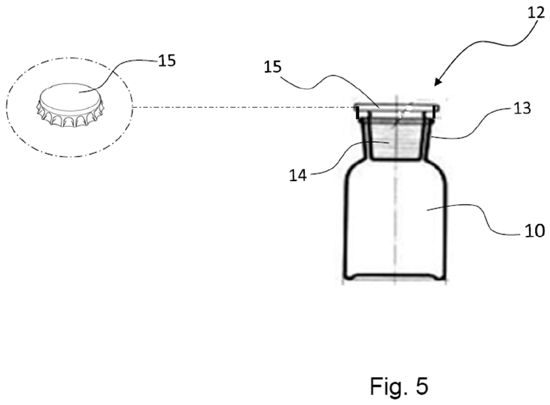

In this context, it should be observed that although the bottles or flasks 10 may have different dimensions (e.g. 10, 30, 50, 70, 100 cl or other) depending on the type of production, on the products - e.g. for home or personal use -, on the process and other parameters, it will still be preferable for the bottles or flasks to have a short neck 13 in relation to their overall height. A proportion like the one shown in figure 5, where the neck is about 20%-25% of the height of the entire bottle, is appropriate.

These proportions advantageously allow a good filling of the case 20, while ensuring the passage of water between the bottles 10.

In accordance with a possible variant of the invention, the bottles 10 are arranged in smaller baskets that are inside the larger case structure 20; this allows to optimize the filling of the spaces, the movement of the bottles and the natural interlocking on one side of the bottle, which is however always in movement on its own axis.

**In** the cosmetics manufacturing process according to the invention, the flasks or bottles 10 that must be lowered into the sea, are housed in a case 20 with a cage-like structure and internally compartmentalized into cells 22 for the bottles 10.

The structure of the case 20 can be made of metal or other appropriate material and its side walls 24, lower base 25 and upper top 26 are open so as to let water pass through; to this end, the structure of the case 20 is predominantly formed with a lattice of bars or rods, while the cells 22 (with the bottles inside them) are staggered from each other to facilitate the flow of water.

The case 20 is internally equipped with multiple planes or levels L1, L2, L3... etc. on which the cells 22 are located: the bottles 10 are housed in their respective cells 22 with a certain slack, so that they can be agitated by the flows that cross the case.

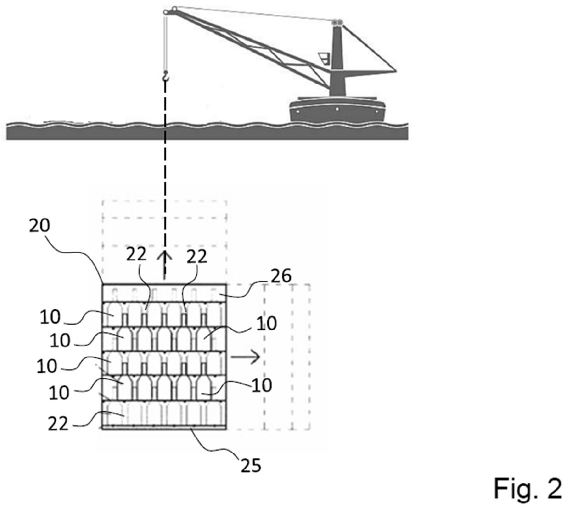

The case 20 prepared with the bottles 10 inside is then lowered into the sea from a vessel as shown in figure 2, placing it at a depth that can vary according to various parameters, such as the water temperature, the type of product that must undergo refining phase, the time required for the conclusion of the production cycle and anything else.

It should be noted that the water temperature can vary depending on the season (i.e. summer or winter), the currents, the site where the refining phase takes place (e.g. Mediterranean Sea or ocean or a sea at northern latitudes, etc.) and therefore the installation depth of the cage 20 can also vary.

The cage can be placed on a seabed or maintained at a certain height from it, also depending on the temperature of the process phase; for this purpose, anchoring systems for the case 20 can be provided, with tie rods and floats on the surface, to sling the cage and keep it suspended at the desired depth.

In accordance with the method of the invention, it is also provided that the case 20 can be inclined or also rotated by 90° with respect to the initial condition with which it is lowered into the water, as graphically illustrated by the arrows and dashed lines in the figures.

The maneuver to rotate the case 20 can preferably be carried out in the water from the vessel, proceeding gradually so as not to damage or excessively stress the bottles 10 which can thus be arranged horizontally; the presence of water between the bottles prevents sudden impacts between them or with the structure and in any case dampens any vibrations induced by the inevitable settlements due to the rotations of the case 20.

The latter allow to obtain a greater mixing of the product inside the bottles, providing for a greater uniformity of the refining process and reducing sediment deposits on the bottom of the bottles 10.

In these circumstances, the permanence duration of the case in an initial or rotated condition will depend on various factors and process parameters mentioned above.

Preferably, the permanence times will be at least one week and even more preferably three or four weeks; however, longer permanence times, even of several months, can be set.

This will depend on the cosmetic substances being processed, their processing process and the phase of the process to be carried out in the submarine environment, such as for example maceration, fermentation, refining or other.

Regardless of this, it should be noted that because of the open structure (cage-like) of the case 20, water can pass through it and lap the bottles, exerting a light agitation action on them due to vibrations of the structure or movements of the bottles within their respective compartments 22.

From what has been described so far, it is possible to understand how the method according to the invention solves the technical problem underlying it, thereby resulting sustainable especially in relation to the current state of the art.

Indeed, the phase of refining, or aging, seasoning, stabilization, mixing, infusion, maturation, maceration, or similar, during which the product is kept in pre-set environmental conditions for a prolonged time, is carried out in a natural way without complex equipment that requires a power supply with electrical energy.

This phase is carried out in a natural way and for a large number of bottles and flasks simultaneously; in this regard, it just matters to be observed that the process can be carried out with a greater number of cases than the single one shown in the drawings and therefore for any quantity of bottled product.

This makes the method of the invention feasible on a large scale (i.e. "scalable") without particular problems, since by exploiting a virtually unlimited environment like the sea, there are in principle no technical or energy drawbacks that could hinder its implementation; moreover, by increasing the scale of the process, advantageous industrial economies can be obtained, making the process favorably usable under this profile.

The applicants are proceeding with in-situ experimental tests in the Italian seas, on some of the substances used in the cosmetic sector and considered suitable for the process of the invention. These are natural raw materials of both vegetable and animal origin, and synthetic raw materials.

### VEGETABLE-DERIVED RAW MATERIALS

The odorous vegetable raw materials are constituted by essential oils that are present in the flowers, roots, roots, rhizomes, leaves, stem, fruits, peels, seeds, bark and wood of trees and in the secretion of various plants. To obtain essential oils, different production methods are used: maceration, infusion, steam distillation, carbon dioxide distillation, pressing, extraction with solvent. After extraction, the vegetable raw materials are oily, liquid, volatile substances with a characteristic odor. They usually retain the name of the plant from which they come. At room temperature, they are almost all liquid; others have a butter or waxy consistency. They are mixtures of various organic substances, and their composition depends on the species of the plant, the type of soil on which it grew, the climate and can also vary depending on the country of origin and the extraction method. They can contain impurities and odorless or weakly odorous parts and can be purified, deterpenated, distilled, rectified.

The known essential oils are very numerous, but those of interest for the present invention are reduced to a few dozen.

Specifically, we have:
- FLOWERS (some examples Cloves, Jasmine, Lily of the valley, Neroli (bitter orange flowers), Rose, Tuberose Ylang ylang, etc.)
- LEAVES (some examples Basil, Geranium, Lavender, Peppermint, Patchouli, Petit-grain, Rosemary, Violet leaves, etc.)
- FRUITS (some examples Orange, Bergamot, Lemon, Vanilla, etc.)
- RESINS (some examples Agarwood, Galbanum, Incense, etc.)
- ROOTS (some examples Iris, Vetiver, etc.)
- MOSSES (some examples Oakmoss, etc.)
- SEEDS (some examples Cardamom, Tonka bean, etc.)
- WOODS (some examples Cinnamon, barks Sandalwood, Guaiac wood, Cedarwood, etc.) ANIMAL RAW MATERIALS

Animal raw materials are composed of extracts from the scent glands of the Moschus, the civet (or African civet) and the beaver. To these is added ambergris. It is an intestinal calculus that the sperm whale gets rid of and that becomes, after floating for years on the sea waters, the raw material used in perfumery. Animal raw materials have been used in the past as base notes to fix the other raw materials. Today they have been excellently replaced by synthetic ones. The main ones are:
- CIVET
- TONKIN MUSK
- AMBERGRIS
- CASTOREUM

### SYNTHETIC RAW MATERIALS

Aromatic chemical research produces a great number of synthetic odorous compounds, some also partly derived from natural molecules, transformed after being extracted from vegetable raw materials - such as castor oil and pine turpentine - or from other raw materials, such as citronella, lemongrass, litseacubeba, geranium etc. Moreover, certain flowers are too delicate to be treated and only the use and association of synthetic products allow the use of the fragrances of lilac, lily of the valley and hyacinth.

For the present invention, the following synthetic substances have been selected:
- Amber, examples of odorous molecules AMBRETTOLIDE, AMBROXAN;
- Musky, examples of odorous molecules: CASHMERAN, GALAXOLIDE;
- Floral, examples of odorous molecules: HELIONAL, HEDIONE, DIHYDROMYRCENOL, IONONE ALPHA, LINALOOL, JASMONE CIS, DAMASCONE ALPHA, PHENYL ETHYL ALCOHOL (PEA), CITRONELLOL LAEVO, CIS-3-HEXENYL SALICYLATE, BENZYL SALICYLATE;
- Fruity, examples of odorous molecules: ALLYL AMYL GLYCOLATE, GAMMA UNDECALACTONE, LIMONENE;
- Woody, examples of odorous molecules: ISO E SUPER, BACDANOL, TRIMOFIX, EVERNYL, VETIVEROL;
- Aldehydic, examples of odorous molecules: ALDEHYDE C12 MNA, ALDEHYDE C12 LAURIC, ALDEHYDE C 11 UNDECYLENIC;
- Spicy, examples of odorous molecules: EUGENOL;
- Animalic, examples of odorous molecules: INDOLE;
- Marine, examples of odorous molecules: CALONE;
- Green, examples of odorous molecules: CIS-3-HEXENOL, TRIPLAL, CYCLOGALBANATE;
- Sweet, examples of odorous molecules: COUMARIN, ETHYL MALTOL, HELIOTROPIN, VANILLIN.

In general, it can be said that the substances that contribute to forming the perfumes for personal and home use considered herein have chemical-physical features such as color, pH, density, etc. but also composition (i.e. mixture) of the ingredients, with a prevalence of odorous molecules, some of which are synthesized specifically for this purpose, for example the family of aldehydes.

These and other characteristics of the invention are stated in the claims that follow.

## Claims

1. A method for manufacturing cosmetic and/or home care products, wherein a perfume, an essence or a composition thereof with an aqueous, alcoholic, hydroalcoholic or oily base, is obtained by mixing various ingredients according to preset formulations and wherein the product or composition is subsequently subjected to a phase of maceration and/or refining and/or aging and/or stabilization and/or maturation carried out at least in part in bottles, flasks or similar containers (10), **characterized in that** said phase of maceration and/or refining and/or aging and/or stabilization and/or maturation is carried out at least in part by immersing the bottles (10) in water, preferably sea water, lake, river or similar.

2. A method according to claim 1, wherein said phase of refining and/or aging and/or stabilization and/or maturation by immersion is carried out by immersing the bottles (10) at depths suitable for obtaining preset temperature and/or pressure conditions.

3. A method according to claims 1 or 2, comprising at least one phase of agitation of the bottles (10) in immersion.

4. A method according to claim 3, wherein the agitation of the bottles (10) is obtained at least in part by means of currents of the immersion water.

5. A method according to any one of the preceding claims, wherein during said phase of refining by immersion the bottles (10) are inclined and/or rotated with respect to an initial immersion condition, for the agitation and/or mixing of the content.

6. A method according to any one of the preceding claims, wherein the bottles (10) are immersed in the sea being arranged in a case (20) comprising a substantially open structure for the passage of water.

7. A method according to claim 6, wherein the case (20) is rotated for the agitation and/or mixing of the content of the bottles (10).

8. A method according to any one of the preceding claims, wherein the perfume, the essence or their composition with an aqueous, alcoholic, hydroalcoholic or oily base comprise raw materials of vegetable origin, including one or more of the following:
• FLOWERS (some examples Cloves, Jasmine, Lily of the valley, Neroli (bitter orange flowers), Rose, Tuberose Ylang ylang, etc.)
• LEAVES (some examples Basil, Geranium, Lavender, Peppermint, Patchouli, Petit-grain, Rosemary, Violet leaves, etc.)
• FRUITS (some examples Orange, Bergamot, Lemon, Vanilla, etc.)
• RESINS (some examples Agarwood, Galbanum, Incense, etc.)
• ROOTS (some examples Iris, Vetiver, etc.)
• MOSSES (some examples Oakmoss, etc.)
• SEEDS (some examples Cardamom, Tonka bean, etc.)
• WOODS (some examples Cinnamon, barks Sandalwood, Guaiac wood, Cedarwood, etc.)

9. A method according to any one of the preceding claims, wherein the perfume, the essence or their composition with an aqueous, alcoholic, hydroalcoholic or oily base comprise raw materials of animal origin, including one or more of the following:
• CIVET
• TONKIN MUSK
• AMBERGRIS
• CASTOREUM

10. A method according to any one of the preceding claims, wherein the perfume, the essence or their composition with an aqueous, alcoholic, hydroalcoholic or oily base comprise synthetic molecules, including one or more of the following:
• Amber, examples of odorous molecules AMBRETTOLIDE, AMBROXAN;
• Musky, examples of odorous molecules: CASHMERAN, GALAXOLIDE;
• Floral, examples of odorous molecules: HELIONAL, HEDIONE, DIHYDROMYRCENOL, IONONE ALPHA, LINALOOL, JASMONE CIS, DAMASCONE ALPHA, PHENYL ETHYL ALCOHOL (PEA), CITRONELLOL LAEVO, CIS-3-HEXENYL SALICYLATE, BENZYL SALICYLATE;
• Fruity, examples of odorous molecules: ALLYL AMYL GLYCOLATE, GAMMA UNDECALACTONE, LIMONENE;
• Woody, examples of odorous molecules: ISO E SUPER, BACDANOL, TRIMOFIX, EVERNYL, VETIVEROL;
• Aldehydic, examples of odorous molecules: ALDEHYDE C12 MNA, ALDEHYDE C12 LAURIC, ALDEHYDE C11 UNDECYLENIC;
• Spicy, examples of odorous molecules: EUGENOL;
• Animalic, examples of odorous molecules: INDOLE;
• Marine, examples of odorous molecules: CALONE;
• Green, examples of odorous molecules: CIS-3-HEXENOL, TRIPLAL, CYCLOGALBANATE;
• Sweet, examples of odorous molecules: COUMARIN, ETHYL MALTOL, HELIOTROPIN, VANILLIN.

11. A case for carrying out the method according to any one of the preceding claims.

12. A case according to claim 11, comprising a plurality of compartments (22) wherein respective bottles (10) are housed.

13. A case according to claims 11 or 12, comprising a substantially cage-like structure.

14. A cosmetic and/or home care product, comprising a perfume, an essence or a composition with an aqueous, alcoholic, hydroalcoholic or oily base comprising a perfume, an essence or similar, refined and/or aged and/or stabilized and/or matured in bottles, flasks or similar (10) immersed in the sea.

15. A product according to claim 14, comprising traces of marine deposits or incrustations on the bottle or flask (10) in which it is contained.
